# EUROPEAN PATENT SPECIFICATION

(11) **EP 3 149 432 B1**
(45) Date of publication and mention of the grant of the patent: **20.02.2019**
(21) Application number: 15732062.3
(22) Date of filing: 28.05.2015
(51) Int. Cl.: G01D 5/353, G01N 21/77

(54) **A FIBER BRAGG GRATING OPTICAL SENSOR HAVING A NANOPOROUS COATING**
OPTISCHER FASER-BRAGG-GITTERSENSOR MIT NANOPORÖSER BESCHICHTUNG
CAPTEUR OPTIQUE À RÉSEAU DE BRAGG SUR FIBRE COMPORTANT UN REVÊTEMENT NANOPOREUX

(30) Priority: 28.05.2014 EP 14170289
(43) Date of publication of application: 05.04.2017
(73) Proprietor: Nederlandse Organisatie voor toegepast- natuurwetenschappelijk onderzoek TNO, 2595 DA 's-Gravenhage (NL)
(72) Inventor: BOERSMA, Arjen, NL-2595 DA 's-Gravenhage (NL); SNELDERS, Dennis Johannes Maria, NL-2595 DA 's-Gravenhage (NL)
(74) Representative: V.O.
(86) International application number: PCT/NL2015/050382
(87) International publication number: WO 2015/183090

(56) References cited:
- WO-A1-2008/125686
- US-A1- 2004 173 004
- US-A1- 2010 247 027
- US-A1- 2010 259 752

## Description

The invention relates to a waveguide comprising a grating and a sensor material, to a method of preparing the waveguide, to a sensor system comprising the waveguide, and to the use of the sensor system for detecting a chemical substance. A chemical substance that is to be detected may hereinafter be referred to as 'analyte'.

Optical sensors are sensors of which the sensing principle and optionally the data transfer make use of electromagnetic radiation. Accordingly, optical sensors have a number of advantages over electronic detection systems. Optical sensors are for example more reliable in environments that are difficult to access and/or hazardous to humans, environments such as those found in the oil and gas industry. Optical sensors are usually not adversely affected by the electromagnetic radiation that is generally produced in for example power cable systems, induction furnaces or equipment for nuclear magnetic resonance measurements, such as MRI or NMR equipment. Other advantages are the easy operation of optical sensors on large distances, their small size, their flexibility and/or the possibility to make a sensor system comprising an array of discrete sensors that all may be read separately from a single optical fibre (a multiplexed sensor system).

Typical sensors that are based on waveguide grating are, *e*.*g*., described in detail in US 5 380 995, US 5 402 231, US 5 592 965, US 5 841 131, US 6 144 026, US 2005/0105841, US 7 038 190, US 2003/156287.

One principle on which such sensors may be based is an axial strain of the waveguide, as a result of an environmental effect that is to be detected, for example by using a coating on the waveguide that deforms under the influence of the environmental effect. An important method via which (a change in) axial strain of the waveguide becomes detectable is to use a grating in the waveguide, such as a Fibre Bragg Grating (FBG). When such a grating, guiding a specific spectrum of electromagnetic radiation, stretches or shrinks under the axial strain, the spectral pattern of transmitted radiation and/or the spectral pattern of reflected radiation (*i.e.* the spectral response) changes. Such changes in the spectral response provide - when measured - quantitative information on the environmental effect. A Fibre Bragg Grating (FBG) is an advantageous type of grating in optical sensors.

The modulation of the waveguide is characterized by a constant periodicity. FBG act as selective mirrors in wavelength, reflecting some wavelengths around the Bragg wavelength, which equals two times the effective refractive index of the waveguide times the periodicity of the refractive index modulation. These parameters are sensitive to external perturbations such as temperature and axial mechanical strain that affect the fibre containing the grating. The result is a shift, without modification of the shape, of the reflected and transmitted spectra. Hence, the information about the external perturbation is wavelength- encoded in the reflected and transmitted spectra. Wavelengths on either side of the Bragg wavelength are transmitted unmodified through the sensor. This property enables multiplexing of many sensors along a single optical fibre and gives birth to quasi-distributed sensing: the optical source and the receiver may be shared between all the sensor units.

Gas sensors using fibre Bragg gratings have been implemented by depositing a sensitive layer on the grating. The layer has the property to expand in presence of the gas to be detected, leading to an axial strain measured by the grating. For example, polyacrylate coatings have been used to measure humidity levels or polyimide coatings have been used to measure CO₂ levels. The usability of these organic polymers is limited though, especially at elevated temperatures.

Palladium coatings have been used to detect hydrogen concentrations. However uniform fibre Bragg gratings covered with Pd have the disadvantage of a quite long response time leading to a hysteresis effect between the responses obtained for increasing and decreasing hydrogen concentrations. Furthermore, the behaviour of such sensors in oxidising (e.g. air) environments is not known since all previous studies showed results of experiments conducted in nitrogen environment and mainly dedicated to aerospace applications.

WO 2008/125686 relates to a multi-use gas sensor wherein an optical fibre having a temperature sensitive detection zone, e.g. a FBG and an associated reaction layer being adapted to generate a temperature change when brought into contact with a gas to be sensed. The inventors consider that the temperature change causes the material in which the grating is present to expand of shrink, dependent on the thermal expansion coefficient of this material The gas reacts with the reaction layer, increasing the temperature (exothermic reaction) or decreasing the temperature (endothermic reaction). This is a destructive detection method, whereby the gas to be sensed is consumed. Moreover, this sensor system requires an elaborated mixture of a catalytic material and a matrix. Furthermore, catalytic materials are known for their limited lifetime, for instance as a result of catalyst decomposition or catalyst poisoning. Hereby properties such as the sensor's sensitivity or detectability are adversely affected, eventually to the extent that the sensor stops working.

WO 2010/074569 relates to a sensor system wherein a FBG is coated with a specific polymer having aromatic groups and analyte-sensitive functional groups. Although this system is suitable for detection of several analytes under extreme conditions, such as higher temperature and pressure, there is a need for alternatives that can operate for a prolonged duration at high temperature and/or that have one or more other favourable properties, such as increased selectivity towards a specific analyte, such as an alkane relative to other chemical substances, e.g. carbon dioxide and/or water.

US2010/247027 relates to an optical fiber sensor with a Fiber Bragg Grating and a nonoporous cladding used for gas detection.

Although it has been widely recognized that optical sensors have a number of advantages over electronic measuring systems, the full potential of optical sensors has not yet been realized. In particular, there is a need for improved sensors for use under extreme conditions, for example under high pressure and/or high temperature. Examples of extreme conditions are conditions that may exist in underground oil or gas reservoirs, or in the equipment that is used to produce oil or gas from these reservoirs. It would in particular be desired to provide an improved sensor for the detection of compounds such as alkanes and alkanols., e.g. under the conditions as mentioned above.

It is an object of the present invention to provide a novel sensor system.

It is also an object of the present invention to provide a novel waveguide comprising a grating, which waveguide can be used in an optical sensor.

It is in particular an object of the invention to provide a novel waveguide that is suitable for use under extreme conditions, such as under conditions that may exist in underground oil or gas reservoirs or in the equipment that is used to produce oil or gas from these reservoirs.

It is a further object of the invention to provide a novel waveguide that is improved, in particular in that a detection system comprising the waveguide is improved in that it offers at least one of the following advantages: a higher selectivity towards an analyte of interest, a larger dynamic range, a higher accuracy, a higher robustness, a lower detection limit, a higher sensitivity and a longer durable (improved functional life time, i.e. at which it maintains satisfactory sensor properties).

The selectivity of a detection system for measuring a certain environmental condition is the extent to which the detector specifically reacts to a change in a selected environmental condition, without being affected by a change in other conditions.

The dynamic range of a sensor system is the range of a changeable quantity that can be measured with that sensor system, the limits of which range are defined by the smallest and the largest value of the changeable quantity that can be measured with that sensor system.

The accuracy of a detection system is the closeness of a reading or indication of that detection system to the actual value of the quantity being measured.

Robustness is the extent to which a detection system is resistant to changes in the detection system, influences from a specific sample and influences from the environment other than the condition, other than the changes in the condition to be measured. Accordingly, as a system is more stable, the back ground noise will be less and/or fewer artefacts will occur in the measuring signal, such a spikes, base line drift and/or base line shifts.

The detection limit is the lowest measurable change in a environmental condition. It is determined by the signal to noise ratio. In general, the detection limit for a particular substance is set at a signal to noise ratio of 2 (if the noise is represented as peak to peak) or 4 (if the noise is represented as the root of the mean square noise (RMS noise)).

The sensitivity of a detection system is the smallest change in a environmental condition, such as a physical or chemical parameter, that can be detected by the detection system.

It has now been found that one or more of these objects are realised by providing a waveguide having a coating which comprises a specific inorganic material respectively a sensor system comprising such a waveguide.

Accordingly, the invention relates to an optical waveguide having a grating, in particular a Fibre Bragg Grating (FBG) which is provided with a coating comprising a nanoporous sensor material, as defined in claim 6.

The sensor material is typically adapted to the waveguide, in a manner allowing contact with a space wherein an analyte of interest may be present and allowing a change in the grating, when the sensor material is in contact with the analyte. In general, the grating is present in the core of the waveguide, the coating at least substantially surrounds the grating and the coating is expandable or shrinkable under the influence of the chemical substance, thereby causing a change in axial strain in the grating when the sensor material is exposed to the chemical substance. This change is detectible by a optical detection unit.

The invention further relates to an optical sensor system for measuring a chemical substance, as defined in claim 1.

The invention further relates to a method for making (manufacturing) an optical waveguide according to the invention, as defined in claim 9.

The invention further relates to the use of an optical sensor system according to the invention for detecting a chemical substance, as defined in claim 13. Such use generally comprises a method for detecting the chemical substance, wherein the sensor material is contacted with a medium (typically fluid; gas/vapour/liquid) in which the chemical substance may be present and detecting a change in an optical property of the grating (as a result of swelling or shrinking of the sensor material when exposed to the chemical substance, and a change in axial strain in the grating as a consequence thereof).

### BRIEF DESCRIPTION OF FIGURES:

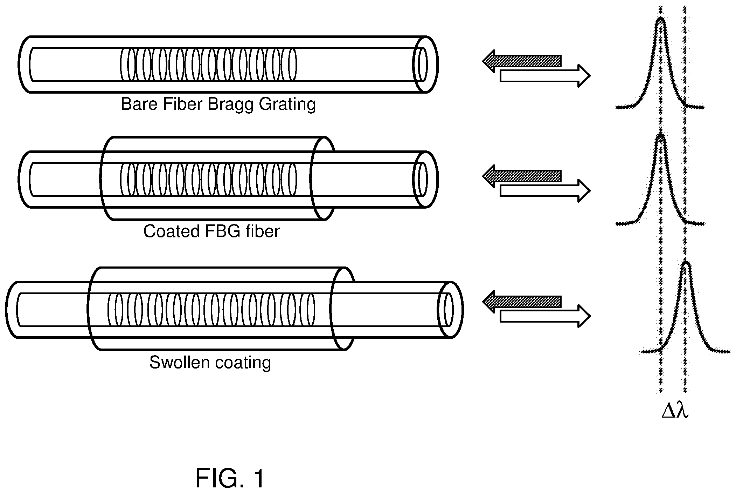

Figure 1: Working principle of a Fibre Bragg Grating based chemical sensor.
Figure 2: Optical microscopy image (magnification 20x) of a zeolite-coated fibre.
Figure 3: Optical microscopy image (magnification 50x) of a zeolite-coated fibre.
Figure 4. SEM image of a fibre coated with 7 µm zeolite coating (entry 1 in Table 1).
Figure 5. SEM image of a fibre coated with 1.2 □m zeolite coating (entry 5 in Table 1).
Figure 6. X-ray diffraction pattern of synthesized crystals.
Figure 7. FBG responses measured for exposure to gases for fibres coated with an MFI zeolite (silicalite).
Figure 8. Response curve measured for exposure of fibre B (12 µm zeolite coating) to C₂H₆
Figure 9. FBG response as a function of the concentration of C₃H₈ in N₂.
Figure 10. FBG response for waveguide coated with silicalite sensor material or ZSM-5 sensor material (non-acidic).

In a preferred embodiment, a sensor system according to the invention is used under extreme conditions, such as under conditions that may exist in underground oil or gas reservoirs, or in the equipment that is used to produce oil or gas from these reservoirs.

The invention is advantageous in that it is possible to provide a coating that can reversibly absorb an analyte of interest in order to perform a continuous measurement of the presence of the analyte. With a continuous measurement is meant a measurement in a non-cumulative way. For example, in a continuous measurement it is possible to measure fluctuations of an environmental effect, such as fluctuations in the concentration of a certain chemical. This is in contrast to a cumulative way of measuring, wherein the total amount of the chemical is observed (like in a dosimeter), i.e. only one or more increases can be observed.

In the field of oil exploration and in the field of gas exploration, it is highly preferred to monitor the downhole environment for a long period of time without replacing the sensor system. In such applications, it is advantageous to use a sensor system according to the invention, because such a system can perform continuous measurements and has a high resistance against the conditions that may be present in downhole environments such as oil wells or gas wells.

A sensor system according to the invention may in particular be used for detecting at least one analyte selected from the group of alkanes, alkanones, ethers and alkanols. The alkane, alkanone, ether or alkanol preferably has 1-12 carbons, more preferably 2-12 carbon atoms, in particular 3-10 carbon atoms, more in particular 4-8 carbon atoms. Preferably the alkane, alkanone, ether or alkanol is a linear compound (having an unbranched carbon chain. A sensor according to the invention is amongst other particular suitable for the detection of methane, ethane or propane.

In a further embodiment a sensor according to the invention is for use in the detection of another analyte, which may be found in an oil/gas well, such as an inorganic analyte selected from the group of water, CO₂, H₂S, H⁺ (pH), CS₂. Good results have been achieved with CO₂

In a specific embodiment, the sensor is used for detecting a small organic tracer (such as a volatile organic) in a oil or gas well.

The sensor system may be used for detection of an analyte in a liquid or gas phase.

Preferably, a sensor system according to the invention comprises a source for providing electromagnetic radiation and a photo-detector.

The term "or" as used herein means "and/or" unless specified otherwise.

The term "a" or "an" as used herein means "at least one" unless specified otherwise.

The term "substantially)" or "essential(ly)" is generally used herein to indicate that it has the general character or function of that which is specified, for instance when referring to essentially spherical it means that it has at least the general appearance of a sphere. When referring to a quantifiable feature, these terms are in particular used to indicate that it is for more than 50 %, in particular at least 75 %, more in particular at least 90 %, even more in particular at least 95 % of the maximum that feature. When referring to an amount-related feature, the amount in terms of weight is meant, unless specified otherwise,

When referring to a "noun" (*e.g.* a compound, an additive *etc.*) in singular, the plural is meant to be included, unless specified otherwise.

For the purpose of clarity and a concise description features are described herein as part of the same or separate embodiments, however, it will be appreciated that the scope of the invention may include embodiments having combinations of all or some of the features described

In the present disclosure, the term 'waveguide' is used for optical waveguides. An optical waveguide is a physical structure that guides electromagnetic waves in at least part of the optical spectrum, *i.e.* in at least part of the spectrum formed by the infrared, visible and ultraviolet ranges of the electromagnetic spectrum.

Usually, a waveguide is of elongate form. In general, a waveguide is cylindrical, in particular with a circular cross-section. A waveguide generally comprises an assembly of a core (through which the electromagnetic radiation propagates) and a cladding covering the core. The core as well as the cladding usually have a substantially circular cross-section. The centre of the cross-section of the cladding usually coincides with the centre of the cross-section of the core. With a cross-section of the waveguide is meant a section through the waveguide that is a plane that is perpendicular to the longitudinal direction of the waveguide.

In a specific embodiment, the cross-section of the core and/or the cladding is different. In particular such waveguide comprises a core and a cladding at least partially covering the core, wherein, in a cross-sectional plane that is perpendicular to the longitudinal direction of the waveguide, the thickness of the cladding in a first radial direction in said plane is different from the thickness of the cladding in a second radial direction in said plane . Such waveguide is described in WO 2010/074569, in particular page 16, line 4 till page 19, line 10.

Common types of waveguides include optical fibres, *e.g.* as referred to in the above cited prior art, and rectangular waveguides. Waveguides are commercially obtainable from various sources. Manufacturing and applications can be found in the Encyclopedia of Laser Physics and Technology (http://www.rp-photonics.com/encyclopedia.html).

Other forms of waveguides are ridge waveguides and slab waveguides.

In the present disclosure, with "grating" is meant a periodic variation of the refractive index of waveguide material in a segment of a waveguide core. A grating reflects particular wavelengths of electromagnetic waves and transmits other wavelengths, and can be used as an inline optical filter or as a wavelength-specific reflector.

As indicated above, the coating of the waveguide comprises a nanoporous sensor material. The term 'nanoporous material' is used herein for materials that essentially consist of a regular framework supporting a regular, open-porous structure. The size of the pores is generally 100 nanometers or smaller. Preferably, the sensor material is an inorganic sensor material containing an oxide, such as a silicate. In an advantageous embodiment the nanoporous material is a ceramic material.

Zeolites, crystalline molecular sieves (which may be a zeolite), and metal organic frameworks are preferred nanoporous materials. In particular, good results have been achieved with zeolites. Nanoporous materials can be subdivided into three categories, set out by IUPAC: microporous materials: 0.2-2 nm, mesoporous materials: 2-50 nm, macroporous materials: 50-1000 nm (http://goldbook.iupac.org/M03906.html;http://soldbook.iupac.org/M03853.ht ml). The nanoporous material on a waveguide in accordance with the invention typically is an essentially mesoporous material. According to the invention, the nanoporous material is capable of adsorption of the chemical substance of interest (analyte), whereby the coating swells or shrinks, which change is detectible by the optical detection unit. In particular, by its capability to shrink or swell when adsorbing the analyte, the coating is effective in stretching or contracting the grating. The coating is effective without causing any significant temperature change. Thus, the sensor functions isothermally.

In general, it is preferred that the nanoporous material is essentially free of any catalytic activity, at least with respect to any reaction wherein the analyte would be a reactant, at least under the conditions at which the sensor is used. Thus, the invention offers a nondestructive detection system. In particular it is preferred that the nanoporous material is essentially free of catalytic activity at a temperature in the range of 100-750 °C, in the range of 150-500 °C or in the range of 150-200 °C, at least in the environment to which the sensor material is exposed during use. In a specific embodiment, the nanoporous material is essentially free of catalytic cracking activity with respect to alkanes having 2-10 carbon atoms, at least at temperature of 175 °C, and preferably at a temperature range mentioned previously in this paragraph.

Generally, the sensor material is essentially free of metallic elements (*i.e.* metal elements in oxidation state 0) that are catalytically active. Good results have been achieved with a sensor, wherein the sensor material is essentially free of transitional metals and noble metals in oxidation state 0, more specifically, with a sensor material that is essentially free of metallic elements.

It should be noted though that in a specific embodiment, use may be made of a metal that is not catalytically active, at least not under conditions wherein the sensor is used for detecting an analyte, which metal induces absorption of a gas and causes swelling.

In a preferred embodiment, the nanoporous sensor material is a zeolite. Zeolites are typically mesoporous. Zeolites have a crystal lattice that typically at least substantially consists of TO₄ tetrahedra. Herein O is oxygen and each T independently represents an atom different from O. In classical zeolites each of the T's is independently either a silicon or a aluminium. However, other elements, such and B, Ge, Zn, P, and transition elements, can also be incorporated into the lattice, replacing part of the Si and/or Al. Such structures are also known in the art as crystalline molecular sieves. Thus, suitable nanoporous sensor materials include zeolites essentially consisting of Si and O, such as silicalite; zeolites essentially consisting of Al, Si and O; crystalline molecular sieves with a zeolite structure, comprising not only Si but also one or more other elements incorporated in the crystal lattice, such as silicoaluminophosphates (SAPOs); and crystalline molecular sieves with a zeolite structure wherein the crystal lattice is composed of oxygen with aluminium and an other element, such as aluminophosphates (AlPOs). AlPOs have strictly alternating AlO₂⁻ and PO₂⁺ units, and the framework is neutral, organophilic,and nonacidic. Within the terminology used in the present disclosure, crystalline molecular sieves composed of oxygen-tetrahedra (TO₄) having a zeolite-like crystal structure (such as LTA, MOR, FAU, MFI, etc, see also below) are considered zeolites.

Suitable nanoporous sensor materials, such as zeolites, may be selected amongst those referred to in Scott M. Auerbach, Kathleen A. Carrado, Prabir K. Dutta-Handbook of Zeolite Science and Technology-CRC Press (2003).

In order to avoid the risk of catalytic activity, the zeolite or other nanoporous sensor material is preferably is non-acidic. Various zeolites exist that can be provided in an acidic form or a non-acidic form. In the acidic form generally at least a substantial fraction of the counter ions for the anionic groups at the surface (inside the pores and outer surface) are acidic ions, such as H⁺ ions. In a non-acidic form, the zeolite is essentially free of acidic ions. Herein the counterions are non-acidic cations, such as alkali metal ions, like sodium ions, potassium ions or lithium ions or alkaline earth metal ions, in particular Ca²⁺ . The skilled person will know how to provide a zeolite in non-acidic form, such as by ion-exchange processes.

In an embodiment, the nanoporous material, in particular the zeolite, is of structure type LTA. Preferred examples thereof are Linde A, ZK-4, N-A, alpha, ZK-21, ZK-22, SAPO-42). It is contemplated that an LTA zeolite is in particular useful for the detection of CO₂, H₂O, CH₄, C₂H₆, CH₃OH, CS₂, C2H₂, NH₃, H₂S or CH₃NH₂, preferably for a compound selected from the group of CO₂, H₂O and CH₄.

In an embodiment, the nanoporous material, in particular the zeolite, is of structure type MOR, or which Mordenite is preferred. It is contemplated that an MOR zeolite is in particular useful for the detection of Ne, Ar, Kr, CO, CO₂, H₂O₂, N₂, O₂, SO₂, NOx, HCl, CH₄ or C₂H₆, preferably for a compound selected from the group of SO₂, NOx and CO_{2.}

In an embodiment, the nanoporous material, in particular the zeolite is of structure type FAU. Preferred examples thereof are Faujasite, Zeolite X, Zeolite Y, Linde X, Linde Y, LZ-210, SAPO-37.). It is contemplated that an FAU zeolite is in particular useful for the detection of SF6, i-C₄H₁₀, i-C₅H₁₂, CHCl₃, isopropanol, CCl₄, benzene, toluene, xylene, pyridine, dioxane, naphthalene or quinolone. Preferably, an FAU zeolite is useful for detection of a aromatic compound, such as benzene, toluene or xylene.

In particular, good results have been achieved with a zeolite structure type MFI, of which Silicalite, ZSM-5 (ZSM-5 Na or ZSM-5 K) and NU-4 are preferred. MFI is in particular useful for the detection of a linear alkane or alkanol.

Silicalite is an essentially completely siliceous zeolite. Silicalite is a polymorph of SiO₂ (refractive index 1.39, density 1.76 g cm⁻³) and has a topologic type of tetrahedral framework. This encloses a three-dimensional system of intersecting channels defined by 10-rings wide enough to adsorb molecules up to 0.6 nm diameter.

An MFI zeolite (in non-acidic form), in particular ZSM-5 or silicalite is particularly preferred for use in a sensor for detecting an alkane or an alkanol having at least 2 carbon atoms. Preferably, the number of carbon atoms is 10 or less, in particular 8 or less. In particular, good results have been achieved with a linear alkane or alkanol, such as ethane, propane, n-butane, n-octane and isopropanol. A coating comprising ZSM-5 or Silicalite was found to have a good selectivity for these analytes over e.g. carbon dioxide, water and methane. Also, it was found that - in a liquid system - the coating has a low sensitivity to potentially disturbing factors like high salt concentration and pH changes.

Alternatives for silicalite or ZSM-5 as a nanoporous sensor material in particular include Linde Type A, AlPO₄-5, zeolite X and zeolite Y.In a further advantageous embodiment, the nanoporous material is a metal-organic framework (MOF's). MOF's, also called "hybrid crystallised solids", are coordination compounds with a hybrid inorganic-organic framework comprising metal ions or semi-metal ions and organic ligands coordinated to the metal ions. These materials are organised as mono-, bi- or tri- dimensional networks wherein the metal clusters are linked to each other by spacer ligands in a periodic way. These materials generally have a crystalline structure and are usually porous. MOF's are in particular suitable for their good adsorption properties with respect to a gaseous analyte, for instance H₂, a hydrocarbon gas (such as CH₄) or CO_{2.}

The metal or semi-metal ions generally have a valence of at least +2. Common ligands include the conjugated bases of organic acids, such as bidentate carboxylates (e.g. oxalate, malonate, succinate, glutarate, phtalate, isophtalate, terephtalate), tridentate carboxylates (e.g. citrate, trimesate).

In a specific embodiment, the MOF is represented by the formula MₙOₖXᵢLₚ, wherein
- each M is independently selected from the group of metal and semi-metal ions, in particular selected from the group consisting of Ti⁴⁺, Zr⁴⁺, Mn⁴⁺, Si⁴⁺, Al³⁺, Cr³⁺, V³⁺, Ga³⁺, In³⁺, Mn³⁺, Mn²⁺, Mg²⁺ and combinations thereof;
- m is 1 , 2, 3 or 4, preferably 1 or 3 ;
- k is 0, 1 , 2, 3 or 4, preferably 0 or 1 ;
- i is 0, 1 , 2, 3 or 4, preferably 0 or 1 ;
- p is 1 , 2, 3 or 4, preferably 1 or 3 ;
- O is oxygen
- each X is independently selected from the group of anions, in particular from the group of monovalent anions, more in particular from the group consisting of OH⁻ Cl⁻, F⁻, I⁻, Br⁻, SO₄²⁻, NO₃⁻, ClO₄⁻ PF₆⁻, BF₃⁻, - (COO)ₙ⁻, R¹-(SO₃)ₙ⁻, R¹-PO₃)ₙ⁻, wherein R¹ is selected from the group consisting of hydrogen and hydrocarbons, in particular hydrogen and C1-C12 hydrocarbons, more in particular hydrogen and C1-C12 alkyls, and wherein n is 1 , 2, 3 or 4 ;
- L is a spacer ligand, in particular a spacer ligand comprising a radical R comprising q carboxylate groups (-COO⁻), wherein,q is 1 , 2, 3, 4, 5 or 6, preferably 2, 3 or 4. R may in particular be selected from the group consisting of C1-C12 alkyl, C2-C12 alkene, C2-C12 alkyne, mono- and poly-cyclic C6-C50 aryl, mono- and poly-cyclic C3-C50 heteroaryl and organic radicals comprising a metal material selected from the group consisting of ferrocene, porphyrin, phthalocyanine and Schiff base R^{X1}R^{X2}-C=N-R^{X3}, wherein R^{X1} and R^{X2} are independently selected from the group consisting of hydrogen, C1-C12 alkyl, C2-C12 alkene, C2-C12alkyne and mono- and poly-cyclic C6-C50aryl and wherein RX3 is selected from the group consisting of C1-C12 alkyl, C2-C12alkene, C2-C12 alkyne and mono- and poly-cyclic C6-C50 aryl.

Such MOF's have been described in WO 2009/130251 in particular with respect to the definitions MₙOₖXᵢLₚ, at page 2 line to page 5, line 19. These MOF's may in particular be used for a sensor for detecting a sulphur containing compound.

The MOF may be present in combination with another nanoporous material, in which case it is typically present in a microparticulate form. In such embodiment, the amount of MOF particles in the coating, is usually in the range of 0.1-50 vol%, preferably in the range of 1-25 vol%.

In an embodiment, the coating is composed of microparticles of the nanoporous material. As used herein, microparticles are particles between 0.01 and 100 µm in size, as determinable by scanning emission microscopy (SEM). The waveguide does not need to be fully coated with the sensor material. It is sufficient that an effective coating is provided at the surface near the grating, in particular for a fibre, the coating at least substantially surrounds the grating.

In a further embodiment, the coating is composed of an essentially homogeneous (monolithic) layer.

Preferably, the coating essentially consists of one or more nanoporous materials. This is advantageous in particular when the sensor system should be suitable for use under extreme conditions such as a high pressure and/or a high temperature. Good results have been achieved with a coating essentially consisting of one nanoporous material.

The coating of a waveguide of the present invention is usually resistant to temperatures of 500° C or more.

Thus, a sensor system according to the invention may advantageously be used to detect an analyte under extreme conditions, such as under conditions that may exist in underground oil or gas reservoirs, or in the equipment that is used to produce oil or gas from these reservoirs. Such extreme conditions include high temperature and high pressure. The temperature may be over 50 °C, over 70 °C or even over 100 °C. The temperature of the environment wherein the sensor detects an analyte usually is 500 °C or less. The pressure may be over 50 bar, over 100 bar, *e.g.* up to 400 bar or up to 150 bar (depending on the depth). Although, the sensor may be used at temperatures in excess of 300 °C, the sensor is in particular useful for detecting an analyte at a temperature of 300 °C or less, in particular in the range of 100-300 °C.

The coating usually has a thickness of at least 1.0 µm, in particular of at least 3 µm, preferably of at least 5 µm, more preferably of at least 7 µm. Usually, the thickness is 100 µm or less, preferably 70 µm or less, in particular 50 µm or less. A relatively thin layer is advantageous for a short response time, a relatively thick layer is generally advantageous for a high sensitivity.

In a specific embodiment, a waveguide according to the invention has a barrier layer that is impermeable to hydrogen (gas). The permeability is for example less than 10¹⁵ molecules/s.cm.atm^{1/2}. Such layer is preferably present between the cladding of the waveguide and the zeolite coating, and may protect the waveguide from harmful effects of the hydrogen. For example, such a layer may comprise a material selected from the group of carbon, silicon carbide, silicon nitride and metals.

In a specific embodiment a waveguide according to the invention has a protective layer, protecting the waveguide against the extreme conditions. Such layer is preferably present between the cladding of the waveguide and the nanoporous sensor material, and may protect the waveguide from harmful effects of salt water, high or low pH (e.g. < 2 or > 12). For example, such layer may comprise a material selected from the group of zircona, and titania sol-gel coatings.

A sensor system of the invention is in particular suitable for detecting a gaseous or vaporous analyte or for detecting an analyte in a liquid phase.

In a specific embodiment, a waveguide according to the invention comprises a plurality of gratings, which are typically spatially apart, preferably 2-500, in particular 2-100 gratings. A waveguide having a plurality of gratings may be used in a multiplex detection system, wherein the spatially apart grating may be provided with a coating capable of interacting with the presence of an analyte of which the presence is to be detected. Each grating on the waveguide can be designed in such a way that it creates a spectral response that is unique with respect to the other gratings on the waveguide. This allows, for instance, a single waveguide to be used to measure an chemical substance at a plurality of places. From a change in a specific unique spectral response (measured at one or both of the ends of a waveguide) it will be clear in the vicinity of which grating the analyte concentration has changed. In particular in case different gratings are coated with different sensor materials, adapted to respond towards a change in different environmental effect, this also allows the use of a single waveguide to measure a multitude changes in analyte concentration.

In a specific embodiment, a waveguide according to the invention comprises a multitude of gratings, at least part of which are present as pairs. If desired, the gratings of a pair can be spatially apart. A first grating of each pair may be used to measure (a change) in a certain analyte, and a second grating of each pair is uncoated or is coated with a coating that is insensitive, or at least not sensitive to a measurable extent, to the analyte to be measured with first grating of the pair. The second grating may be used for monitoring the temperature, and may in particular be used to correct for the influence of temperature on the first grating of the pair.

In a further embodiment, a grating is partly coated (in the longitudinal direction of the waveguide) with a coating that is sensitive to a measurable extent to the environmental effect to be measured. The grating is e.g. coated for only about half of its length. Accordingly, the coated part of the grating then in essence forms the first grating and the part of the grating that is not coated with the sensitive coating forms the second grating.

Usually, a waveguide according to the invention comprises an assembly of a core and a cladding. The electromagnetic radiation used for measuring predominantly propagates through the core. The cladding usually encloses the core; it may protect the core, and/or aid in the propagation of radiation through the core.

The invention further relates to a method for making an optical waveguide according to the invention, comprising
- providing an optical waveguide having a Fibre Bragg Grating;
- contacting the surface of the waveguide that is to be provided with a coating comprising a zeolite sensor material with a zeolite synthesis solution comprising a silicon source and a structure directing agent (such as a quaternary ammonium compound);
- depositing zeolite crystals from the zeolite synthesis solution on the surface of the waveguide, thereby forming a coating; and
- calcining the coating.

The zeolite synthesis solution can be a solution already known in the art for preparing the zeolite of interest. For example, the procedure described in Sensors and Actuators B 135 (2009) 420-425 can be used. This publication describes the coating of an LPG sensor with a silicalite film. It should be noted that LPG sensors work in a conceptually different way than FBG, namely by monitoring a change in refractive index. There is no suggestion in this publication that the coating would be capable of expanding or shrinking in the presence of an analyte, such as an alkane, to the extent that it causes a detectible change in axial strain in the waveguide.

Preferred silicon sources are tetraethyl orthosilicate, tetramethyl orthosilicate, colloidal silica sol, fumed silica, sodium metasilicate.

In case, the zeolite is to include aluminium an aluminium source is also provided in the zeolite synthesis solution. Preferred aluminium sources are sodium aluminate, aluminium hydroxide, aluminium isopropoxide, aluminium nitrate, aluminium sulfate, aluminium metal.

Preferred structure directing agents are a quaternary ammonium compounds, in particular tetrapropylammonium hydroxide, tetrapropylammonium bromide, tetramethylammonium hydroxide, tetramethylammonium bromide, tetraethylammonium hydroxide, tetraethylammonium bromide, ethanoltripropylammonium hydroxide, ethanoltripropylammonium bromide.

Calcination can be carried out in a manner known per se, e.g. a temperature in the range of ..300-700°C.

Deposition (crystallization) is suitably carried out at elevated temperature, in particular in the range of 150-250 °C, preferably in the range of 175-220 °C.

Deposition is suitably carried at a pressure in the range of 0-10 bar.

A coating comprising a MOF may be made based on methodology known in the art, for instance based on methodology described in Adv.Mater. 2010, 22, 2695/2688 , in J. AM. CHEM. SOC. 2007, 129, 8054-8055, in Nano Letters 2004, Vol 4, No2, 365/371, in J. AM. CHEM. SOC. 2007, 129, 15118-15119, in J. Mater. Chem. 2007, 17, 2785/2792, or in J Am. Chem Soc 2008, 130, 14404/14405.

In particular, a method according to the invention for preparing a waveguide comprising a coating comprising a MOF, comprises subjecting the surface of the waveguide to a functionalization treatment during which chelating groups or other functional groups capable of binding with a metal ion (for the MOF) are provided on the surface, such as carboxylate groups, thereafter contacting the surface, provided with the functional groups, with a metal-organic framework synthesis solution, and depositing a metal-organic framework from said synthesis solution to the surface of the waveguide.

A preferred functionalisation treatment, comprises silanization of the surface with silane. Suitable silanes include alkenylsilanes, e.g. trichloroalkenyl silanes, with vinyl group, such as 7-octenyl-trichlorosilane. After binding to the surface the vinyl group is converted in a carboxylate group. This can be done in a manner known per se.

The skilled person will know how to deposit a specific MOF based on the information disclosed herein, common general knowledge about forming the specific MOF and the literature cited herein.

The invention will now be illustrated by the following examples.

### EXAMPLES

### Example 1

### General

Tetraorthosilicate and tetrapropylammonium hydroxide (1M solution) were purchased from Sigma Aldrich and used without further purification. Glass fibres (125 µm, SiO2 with Ge-doped core, FBG length 10 mm) were purchased from Fibretronix.

Optical microscopy was performed on a Zeiss Axio Imager M1m microscope and SEM was performed on a Fei Quanta 600 microscope.

### Synthesis of zeolite coating on FBG

A film of completely siliceous zeolite film (silicalite) was grown directly on a 125 µm glass fibre by *in situ* hydrothermal synthesis from a clear precursor solution, according to Zhang, J.; Tang, X.; Dong, J.; Wei, T.; Xiao, H., Sensors and Actuators B, 2009, 135, 420-426. The procedure involved mixing 10.2 mL Tetraethyl orthosilicate (TEOS), 5.6 mL 1M Tetrapropylammonium hydroxide (TPAOH) and 30 mL demineralized water. This mixture was stirred vigorously at 50 °C for 3h and then left to stand at RT for 1-20 days (aging period). The mixture was then diluted by adding an equal volume of demineralized water. Then 50 mL of the diluted solution was poured into a glass tube containing the glass fibre. The glass tube contains an insert that keeps the glass fibre in the center of the tube without touching the walls of the vessel. The fibre was placed such that the FBG segment was completely immersed in the synthesis solution. The glass tube was inserted into a Teflon-lined stainless steel autoclave which was placed vertically in an oven. The synthesis was performed at 180°C under autogeneous pressure for 4-8 h. After the synthesis, the coated glass fibre was rinsed with demineralized water and calcined in air (500°C, 2h, heating rate 250 °C/h). The coated FBG was then welded to a second piece of glass fibre containing a connector.

After each experiment the zeolite layer was characterized by optical microscopy, which allowed for determination of the layer thickness. In several cases, further characterization was done by SEM, which allowed for measurement of the dimensions of the crystallites. (Table 1)

**Table 1. Optimization of zeolite coating on FBG.**

| Entry | Aging time (d) | Synthesis temp (°C) | Synthesis time (h) | Layer thickness (µm)^{a} | Crystallite dimensions | | |
|---|---|---|---|---|---|---|---|
| | | | | | (µm)^{b} length | breadth | thickness |
| 1 | 0 | 180 | 4 | 6.9 | 16 | 5.9 | 1.8 |
| 2 | 5 | 180 | 4 | 11.7 | | | |
| 3 | 10 | 180 | 4 | 6.5 | 15 | n.d.^{c} | 1.2 |
| 4 | 19 | 180 | 4 | 11.8 | | | |
| 5 | 5 | 150 | 4 | 1.2 | 2.9 | 1.9 | 1.0 |
| 6 | 4 | 205 | 4 | 17.7 | 16 | 4.7 | 2.0 |
| 7 | 1 | 180 | 8 | 18.3 | | | |
| 8 | 4 | 180 | 8 | 25.0 | | | |
| 9 | 5 | 180 | 8 | 20.8 | 22 | 7.1 | 2.6 |

| | | | | | | | |
|---|---|---|---|---|---|---|---|
| a). Determined by optical microscopy. b). Determined by SEM. c). not determined. | | | | | | | |

The period in between the preparation of the precursor solution and the actual synthesis of the zeolite film is referred to as the aging period. The effect of the length of this period on the properties of the eventually obtained zeolite film was investigated (Table 1, entries 1-4). In four experiments with aging periods of 0 to 19 days, the layer thickness varied between 6.5 and 11.9 µm. No trend was observed in the dimensions of the crystallites either. Secondly, the influence of the reaction temperature was investigated (entries 2, 5, 6). Lowering the temperature from 180 to 150 °C gave a much thinner layer of only 1.2 µm as well as much smaller crystallites. An increase of the temperature from 180 to 205 °C led to a thicker layer of 17.7 µm while no effect on the crystallite dimensions was observed. Finally, the reaction time was extended from 4 to 8 h, with aging periods varying from 1 to 5 days (entries 7-9). These experiments showed that a longer reaction time produced a thicker layer as well as larger crystallites.

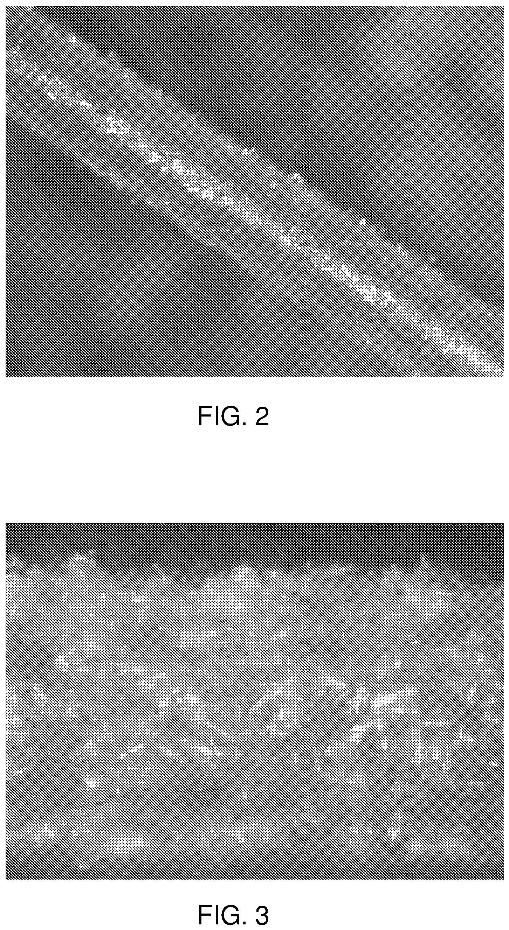

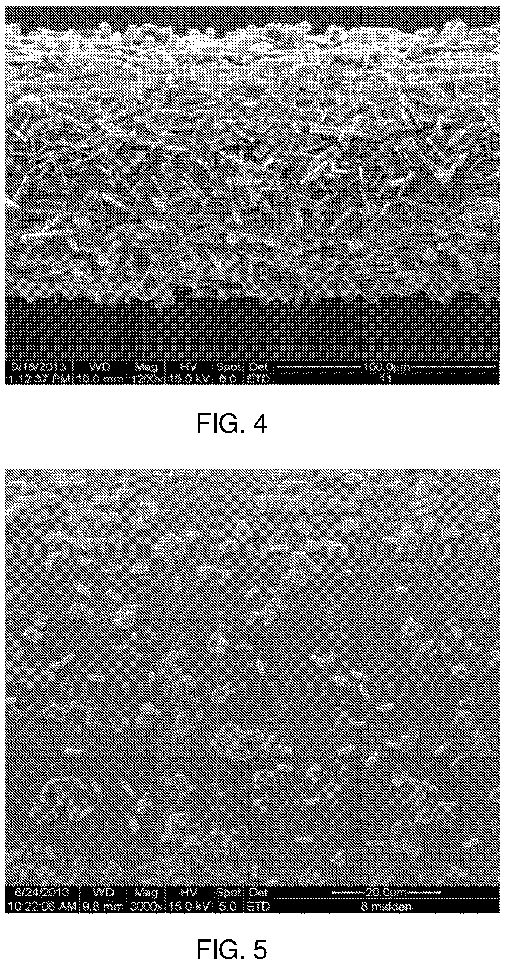

Representative images obtained by optical microscopy are shown in Figure 2 and Figure 3. SEM images from two differently coated fibres are shown in Figure 4 and Figure 5. These images show that a homogeneous layer of randomly deposited crystallites has formed onto the glass fibre surface. In Figure 5, which was taken from a fibre containing a thin layer of small crystals, it can be seen that a continuous layer of intergrown crystals has formed close to the fibre surface. It seems likely that such a continuous layer is also present in the case shown in Figure 4.

In general, it was concluded that the adhesion of the zeolite layer to the glass fibre was quite strong. Detachment of the zeolite layer from the fibre surface was never observed.

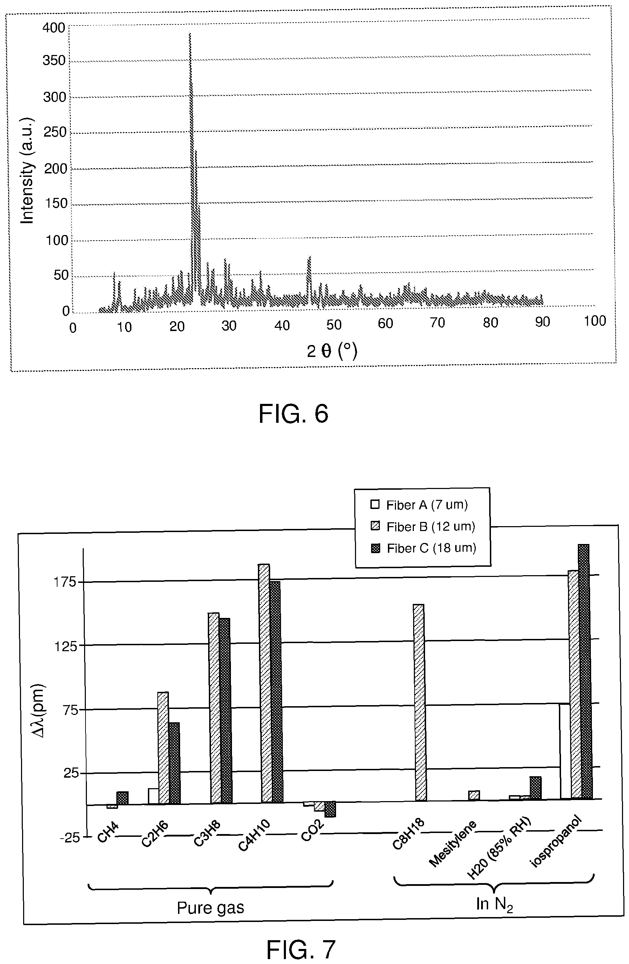

An XRD measurement was performed on zeolite material that was collected from the synthesis vessel (Figure 6).

The XRD spectrum was compared with a spectrum of silicalite as reported in literature (Yu, J. in : Introduction to zeolite science and practice - 3rd Ed., Ch. 3 Synthesis of zeolites, p. 47, 2007, Elsevier, Eds. Cejka, J.; van Bekkum, H.; Corma, A.; Schuth, F.) . The measured diffraction pattern corresponds well with the literature pattern. Characteristic strong reflections are reported at d =11.01, 3.829, 3.806 and 3.698 Å. In the measured spectrum, strong reflections are observed d = 11.08, 3.845, 3.716 and 3.649 Å. The XRD spectrum thus confirms that the synthesized material is silicalite.

### Gas adsorption measurements

Measurements to assess gas adsorption characteristics were performed for three fibres with different zeolite coating thicknesses, *i.e.* 7, 12 and 18 µm. The fibres were inserted into an airtight, custom-made glass measuring chamber. The fibres were connected to an interrogator which analyses the wavelength of light reflected by the FBG. With this setup, it was possible to continuously measure the reflected wavelength as a function of exposure to a certain gas . The glas flow was controlled by a purposely built *Gas Exposure System*, which is equipped with software controlled glass flow meters and a humidity sensor. The measurements were performed at room temperature. The influence of temperature fluctuations was determined by measuring the signal of a bare FBG over a period of time and was found to have a maximum deviation of around 5 pm..

For fibre A (7µm zeolite coating, entry 1 in Table 1), the probe gases were CH₄, C₂H₆, CO₂, H₂O (85% relative humidity in N₂) and isopropanol (prepared by bubbling a stream of N₂ through liquid isopropanol). The data are presented in Figure 7 and Table 2, which show the shift in reflected wavelength (in pm) upon exposure to each of the different gases. In all cases presented in Figure 7, the shift has been considered with respect to the signal observed for pure N₂. The data are averages of several measurements.

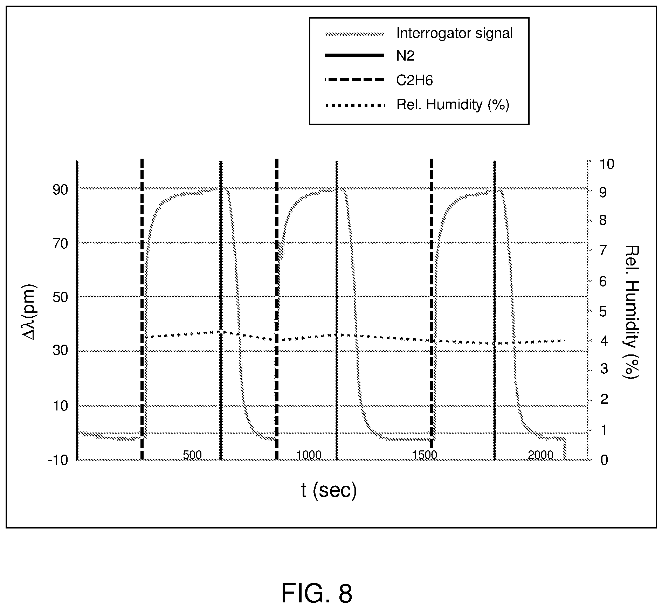

For fibre A, the only two gases that gave a significant response were C₂H₆ and isopropanol. Going to a thicker zeolite layer, *i.e.* fibre B (12 µm), a much larger response was observed for all gases except CH₄ and H₂O, which still barely gave any response. The response curve as measured for exposure of fibre B to C₂H₆ is shown in Figure 8. Upon exposure to C₂H₆, a shift in the reflected wavelength of 90 pm is observed. Upon flushing the system with N₂, the signal returned to its starting value, indicating that the effect is fully reversible. Several repeats of this process showed a good reproducibility.

A positive influence of molecule size was seen between the linear alkanes up to butane. For octane (*i.e.* a N₂ stream saturated with gaseous octane by bubbling through liquid octane), no further increase in the magnitude of the signal was observed compared with butane. This trend may be explained by the critical diameters (CD) of these molecules, which are listed in Table 2. A positive correlation between CD and the magnitude of the signal is apparent. Since all linear alkanes starting from C₃H₈ have equal CDs, these molecules all lead to similar signals. For CO₂, a small but significant negative response was observed, which implies that the zeolite shrinks upon CO₂ exposure.

Isopropanol gave a response that was similar to those of C₃H₈ and C₄H₁₀.. On the other hand, for mesitylene only a negligible response was observed.

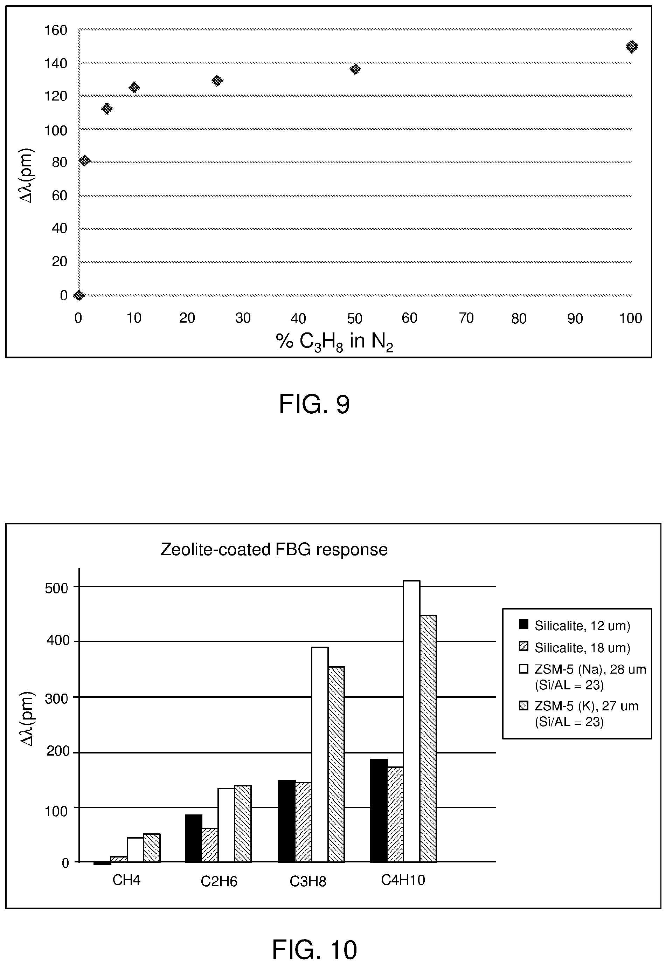

The FBG response as a function of the concentration of C₃H₈ in N₂ was measured for concentrations starting from 1% (Figure 9). These measurements showed a very sharp increase for concentrations lower than 10%, while higher concentrations only lead to a small further increase of the signal. Clearly, the sensor is very sensitive to low concentrations of C₃H₈..

Subsequently, a fibre with an zeolite coating of 18 µm was tested (fibre C). The observed responses for this fibre were in general comparable to those observed for fibre B (Figure 7).

Further measurements were performed in the liquid phase, by immersing the zeolite-coated FBG sensor (measurements only performed for fibre B) in a beaker containing stirred H₂O at room temperature. The data are presented in Table 2. The shift in wavelength upon addition of 1M NaCl as well as to changes in the pH (by addition of either concentrated HCl or KOH solutions) was measured. It could be concluded that the sensor has a very low sensitivity for salt and pH.

**Table 2. FBG responses (Δλ in pm) measured for exposure to gases or liquids, for fibres coated with MFI zeolite.**

| | **Fibre A** | **Fibre B** | **Fibre C** | **CD (Å)** |
|---|---|---|---|---|
| Zeolite layer thickness (µm) | 6.9 | 11.8 | 18.3 | |
| CH₄^{a} | 0 | -1.9 | 10 | 4.0 |
| C₂H₆^{a} | 13 | 88 | 64 | 4.4 |
| C₃H₈^{a} | n.d. | 150 | 145 | 4.9 |
| C₄H₁₀^{a} | n.d. | 187 | 173 | 4.9 |
| CO₂^{a} | -3.0 | -7.6 | -12 | 2.8 |
| C₈H₁₈^{b} | n.d. | 154 | n.d. | 4.9 |
| Mesitylene^{b} | n.d. | 7 | n.d. | 8.4 |
| H₂O (85% RH)^{b} | 2.5 | 2.5 | 19 | 3.2 |
| isopropanol^{b} | 76 | 180 | 215 | |
| 1M NaCl^{c} | n.d. | -4 | n.d. | |
| pH 13^{c} | n.d. | 17 | n.d. | |
| pH 12^{c} | n.d. | 0 | n.d. | |
| pH 2^{c} | n.d. | 2 | n.d. | |
| pH 1^{c} | n.d. | 10 | n.d. | |
| 0.2 vol% decane^{d} | n.d. | 170 | n.d. | 4.9 |

| | | | | |
|---|---|---|---|---|
| n.d. = not determined a). Pure gas (data with respect to N₂ baseline signal). b). In N₂ (data with respect to N₂ baseline signal). c). In H₂O (data with respect to H₂O baseline signal). d) In mesitylene, at 150 °C. (data with respect to mesitylene baseline signal at 150 °C). | | | | |

Finally, in order to assess the temperature stability of the sensor, fibre B was immersed into mesitylene at 150 °C. As was established in previous experiments, mesitylene itself does not lead to a significant response. After the signal had become stable, a small quantity (0.2 vol%) of liquid decane was added to the stirred mixture. Immediately a large increase of the reflected wavelength was observed, indicating that the zeolite-coated FBG still functioned at 150 °C. Adding higher concentrations of decane (tested up to 1.5 vol%) did not lead to a further change in the signal, suggesting that the zeolite coating was saturated after exposure to 0.2 vol% decane. For fibre B, the response times and regeneration times were estimated from the obtained response curves for all gases (Table 3). The response time was defined as the time required until no further change of the signal was observed. Some variation between the different gases was observed and in general, the response times were 300 s or less. The regeneration times were defined as the time required for full recovery of the baseline signal. In several cases the regeneration took longer than the response time. For butane and octane, the regeneration was very slow and was in fact not achieved within the timeframe that was used for the experiment. In those cases, as well as for exposure to liquid decane, heating of the coated segment was required for full regeneration.

**Table 2. Response times and regeneration times for fibre B (12 µm zeolite coating).**

| **Gas** | **response time (s)** | **regeneration time (s)** |
|---|---|---|
| CH₄ | 60 | 60 |
| C₂H₆ | 200 - 300 | 200 |
| C₃H₈ | 150 - 300 | 600 - 800 |
| C₄H₁₀ | 200 | 2000 < |
| C₈H₁₈ | 250 | 4000 < |
| CO₂ | 100 - 200 | 100 - 200 |
| IPA | 100 | 700 |

### Example 2

A coating of a non acidic ZSM-5: was provided on a fibre using similar methodology as for Example 1, except for a different zeolite synthesis solution. This solution contained 19.2 mL Tetraethyl orthosilicate (TEOS), 11.3 mL 1M Tetrapropylammonium hydroxide (TPAOH), 0.460 g NaAlO₂ and 60 mL demineralized water. Of the resulting mixture, an amount of 44 mL was used undiluted.

Figure 10 shows responses for several compounds by silicalite containing ZSM 5 , sodium form (Na) and ZSM 5 potassium form (K) (prepared by ion exchange starting from the Na form). It was found that the response for the ZSM 5 coating was larger.

### Example 3

The performance of the zeolite-coated FBG sensors was evaluated under extreme conditions, *i.e.* at temperatures up to 270 °C and pressures during use as a sensor of up to 200 bar. The gases that were used are CH₄, C₂H₆, C₃H₈ and CO_{2.} Three different zeolite-coated FBGs were tested: Fibre B (coated with 12 µm silicalite; same fibre as mentioned in Table 2), Fibre D (coated with 17 µm silicalite) and Fibre E (coated with 26 µm ZSM-5).

Firstly, the robustness of the fibres was tested by exposing them to 300 bar of liquid H₂O for 1h followed by 200 bar of gaseous N₂ for 1h. In all cases, the fibres withstood the circumstances without any noticeable damage.

Subsequently, measurements were performed by exposing the coated FBGs to N₂ gas at high temperature and/or pressure, followed by exposing them to C₂H₆ or C₃H₈ gas at identical conditions. The difference in the wavelengths observed at N₂ and C₂H₆ or C₃H₈ exposure, represents the measured signal. Table 3 shows the measured data. For CH₄ and CO₂, under all conditions, no significant response was measured for any of the three sensors. For C₂H₆ and C₃H₈, significant responses were measured at low temperature and low pressure, as well as at high temperature and elevated pressure (8 bar or higher). For C₃H₈, measurements were performed at high temperature and high pressure.. Under these circumstances, significant responses were observed for all three sensors, most notably for fibre E.

**Table 3. Shift in observed FBG signal (N₂ used as baseline) for fibres B, D and E.**

| Gas | p (bar) | T (°C) | Δλ (rel. to N2) (pm) | | |
|---|---|---|---|---|---|
| | | | Fibre B 12 µm silicalite | Fibre D 17 µm silicalite | Fibre E 26 µm ZSM-5 |
| CH4 | 0 | 22 | -2 | -2 | -13 |
| CH4 | 0 | 254 | 7 | 6 | 9 |
| CH4 | 20 | 269 | -2 | 5 | 6 |
| | | | | | |
| C2H6 | 0 | 22 | 91 | 61 | 87 |
| C2H6 | 0 | 254 | 15 | 9 | 7 |
| C2H6 | 20 | 279 | 54 | 43 | 58 |
| | | | | | |
| C3H8 | 0 | 22 | 175 | 159 | 244 |
| C3H8 | 0 | 254 | 0 | 4 | 6 |
| C3H8 | 8 | 264 | 51 | 45 | 71 |
| CO2 | 0 | 22 | 9 | 3 | -6 |
| CO2 | 0 | 254 | -21 | -12 | -10 |
| CO2 | 20 | 265 | 3 | 8 | 8 |
| | | | | | |
| 2% C3H8 | 20 | 265 | -14 | -6 | -6 |
| 10% C3H8 | 20 | 260 | 5 | 10 | 19 |
| 25% C3H8 | 17 | 265 | 2 | 6 | 19 |
| | | | | | |
| 10% C3H8 | 200 | 260 | 48 | 65 | 117 |
| 10% C3H8 | 200 | 260 | 49 | 39 | 133 |

### Example 4

Herein, the application of a coating consisting of the MOF HKUST-1 to a FBG is described, using a self assembled monolayer (SAM) approach.

In order to improve the adhesion of the MOF layer to the quartz fibre surface, a self assembled monolayer (SAM) was applied to the fibre surface. A stepwise procedure was used, which allowed for a covalent bonding between the quartz surface and the MOF crystals. The procedure is illustrated by the following reaction scheme:

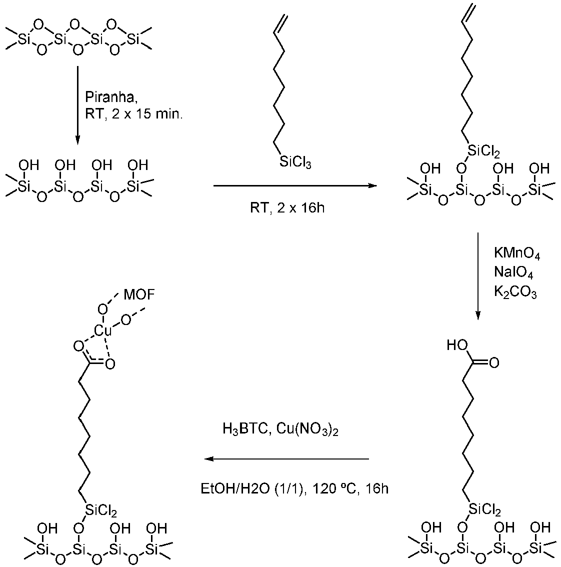

Firstly, the substrates were treated with a piranha (30 % H₂O₂/ conc. H₂SO₄, 1/2 v/v) solution (2 x 15 min), rinsed with demi-water and dried for 2h at 60 °C. Secondly, the substrates were placed in an exsiccator vessel along with an open vial containing 0.5 mL of 7-octenyl-trichlorosilane. The exsiccator vessel was placed under vacuum, closed and left to stand overnight. The treatment with 7-octenyl-trichlorosilane was then repeated once. Finally, the substrates were placed in an aqueous solution containing KMnO₄ (0.5 mM), K₂CO₃ (1.8 mM) and NaIO₄ (19.5 mM) and left to stand overnight at room temperature. The substrates were then rinsed with 0.3 M NaHSO₃, demi-water, 0.1 M HCl, water and ethanol.

The surface pretreated substrates were coated with a MOF layer by *in situ* synthesis. The substrates were immersed in a glass tube containing a clear synthesis solution containing trimesic acid (H3BTC) and Cu(NO₃)₂ in a H₂O/EtOH (1/1 v/v) mixture. The glass tube was then inserted in a stainless steel autoclave, which was placed inside an oven and heated for a certain period. After cooling down, the substrates were removed from the autoclave and analyzed by microscopy.

| **Substrate** | **H₃BTC (M)^{a}** | **Cu(NO₃)₂ (M)^{a}** | **Temp. (°C)** | **Time (h)** | **Layer thickness (µm)^{b}** |
|---|---|---|---|---|---|
| Plates | 0.059 | 0.12 | 120 | 8 | 0-80 |
| Plates | 0.030 | 0.060 | 120 | 8 | Very little deposition |
| Fibre | 0.059 | 0.12 | 120 | 16 | 150-200 |
| Fibre | 0.030 | 0.060 | 120 | 16 | 50-80 |
| Fibre^{c} | 0.030 | 0.060 | 120 | 16 | 80-120 |

| | | | | | |
|---|---|---|---|---|---|
| a) H₃BTC was dissolved in EtOH and Cu(NO₃)₂ in H₂O. These two solutions were mixed. The final concentrations in the synthesis solution (in H₂O/EtOH (1/1 v/v)), after mixing, are listed in this table. b) Analyzed by DEKTAK profilometer for plates and by optical microscopy for fibres. c) A fibre with a fibre bragg grating segment was used. | | | | | |

The coating experiments on pretreated plates generally yielded relatively homogeneous MOF layers of varying thickness depending on the concentration of the synthesis solution and the reaction time. Analysis by microscopy revealed that the MOF crystals were deposited in a random fashion on the surface of the plates), but the surface was not completely covered. Analysis by DEKTAK profilometer shows that the thickness of the crystals reached 80 µm, but bare regions were also present. The adhesion of the crystal layer was relatively strong; scratching with a sharp object was required to remove the layer.

The coating experiments on pretreated fibres (according to the invention) yielded continuous MOF layers with a thickness depending on the concentration of the synthesis solution, the synthesis time and temperature. Heating at 120 °C for 16 h proved a reliable method for achieving a continuous coating with a significant thickness and strong adhesion. In general, a blue layer of MOF crystals was firmly attached to the fibre.

A fibre containing a fibre bragg grating segment was coated with a HKUST-1 layer of 80-120 µm. This fibre was welded to a connector and analysed for gas adsorption characteristics in the *Gas Exposure System*, using a custom-made glass measuring chamber and an interrogator to analyse the reflected wavelength. The MOF-coated fibre reproducibly showed a response to exposure to moisture (in gaseous form in N₂) and to CO₂ and C₃H₈ gas. In all cases, an initial significant response was observed, which shows that MOF layers can be used as coatings for FBGs and that swelling of the MOF layer due to gas adsorption is feasible.

## Claims

1. An optical sensor system for measuring a chemical substance, the sensor system comprising an optical waveguide having a Fibre Bragg Grating, which waveguide is provided with a coating comprising a nanoporous sensor material, the sensor unit further comprising an optical detection unit for detecting a change in an optical property of the waveguide, wherein the grating is present in the core of the waveguide, the coating at least substantially surrounds the grating, which coating is isothermally expandable or shrinkable under the influence of the chemical substance, thereby isothermally causing a change in axial strain in the grating when the sensor material is exposed to the chemical substance, which change is detectible by the optical detection unit.

2. An optical sensor system according to claim 1, wherein the sensor material comprises a non-acidic zeolite, which non-acidic zeolite preferably is selected from the group of non-acidic MFI structure type zeolites, non-acidic LTA structure type zeolites, non-acidic MOR structure type zeolites and non-acidic FAU structure type zeolites.

3. An optical sensor according to claim 2, wherein the non-acidic zeolite is selected from the group of silicalite, ZSM-5, Linde Type A, AlPO₄-5, AlPO₄-34, zeolite X and zeolite Y, preferably selected from the group of ZSM-5 and silicalite.

4. An optical sensor according to any of the preceding claims, wherein the sensor material comprises a metal-organic framework (MOF), in particular a MOF represented by the formula MₙOₖXᵢLₚ, wherein
- each M is independently selected from the group of metal and semi-metal ions, in particular selected from the group consisting of Ti⁴⁺, Zr⁴⁺, Mn⁴⁺, Si⁴⁺, A13+, Cr³⁺, V³⁺, Ga³⁺, In³⁺, Mn³⁺, Mn²⁺, Mg²⁺ and combinations thereof;
- n is 1 , 2, 3 or 4, preferably 1 or 3 ;
- k is 0, 1 , 2, 3 or 4, preferably 0 or 1 ;
- i is 0, 1 , 2, 3 or 4, preferably 0 or 1 ;
- p is 1 , 2, 3 or 4, preferably 1 or 3 ;
- O is oxygen
- each X is independently selected from the group of anions, in particular from the group of monovalent anions, more in particular from the group consisting of OH⁻ Cl⁻, F⁻, I⁻, Br⁻, SO₄²⁻, NO₃⁻, ClO₄⁻ PF₆⁻, BF₃⁻, -(COO)ₙ⁻, R¹-(SO₃)ₙ⁻, R¹-PO₃)ₙ⁻, wherein R¹ is selected from the group consisting of hydrogen and hydrocarbons, in particular hydrogen and C1-C12 hydrocarbons, more in particular hydrogen and C1-C12 alkyls, and wherein n is 1 , 2, 3 or 4 ;
- L is a spacer ligand, in particular a spacer ligand comprising a radical R comprising q carboxylate groups (-COO⁻), wherein,q is 1 , 2, 3, 4, 5 or 6, preferably 2, 3 or 4 . R may in particular be selected from the group consisting of C1-C12 alkyl, C2-C12 alkene, C2-C12 alkyne, mono- and poly-cyclic C6-C50 aryl, mono- and poly-cyclic C3-C50 heteroaryl and organic radicals comprising a metal material selected from the group consisting of ferrocene, porphyrin, phthalocyanine and Schiff base R^{X1}R^{X2}-C=N-R^{X3}, wherein R^{X1} and R^{X2} are independently selected from the group consisting of hydrogen, C1-C12 alkyl, C2-C12 alkene, C2-C12alkyne and mono- and poly-cyclic C6-C50aryl and wherein RX3 is selected from the group consisting of C1-C12 alkyl, C2-C12alkene, C2-C12 alkyne and mono- and poly-cyclic C6-C50 aryl.

5. An optical sensor system according to any of the preceding claims, wherein the coating has a thickness of 1-100 µm, preferably of 3-70 µm, more preferably of 5-50 µm.

6. An optical waveguide having a Fibre Bragg Grating, which waveguide is provided with a coating comprising a nanoporous sensor material, wherein the grating is present in the core of the waveguide, the coating at least substantially surrounds the grating, which coating is isothermally expandable or shrinkable under the influence of a chemical substance, thereby isothermally causing a change in axial strain in the grating when the sensor material is exposed to the chemical substance, which change is detectible by an optical detection unit.

7. Waveguide according to claim 6, wherein the coating comprises a non-acidic zeolite, preferably selected from the group of selected from the group of non-acidic MFI structure type zeolites, non-acidic LTA structure type zeolites, non-acidic MOR structure type zeolites and non-acidic FAU structure type zeolites, in particular a non-acidic zeolite selected from the group of silicalite, ZSM-5, Linde Type A, AlPO₄-5, zeolite X and zeolite Y, more in particular a non-acidic zeolite selected from the group of ZSM-5 and silicalite.

8. An optical waveguide according to claim 6 or 7, wherein the sensor material comprises a metal-organic framework (MOF), in particular a MOF represented by the formula MₙOₖXᵢLₚ as defined in claim 4.

9. A method for making an optical waveguide according to any of the claims 6 or 7, the method comprising providing an optical waveguide having a Fibre Bragg Grating and providing a surface of the optical waveguide with a coating comprising the nanoporous sensor material.

10. A method according to claim 9, wherein the sensor material comprises a zeolite, the method comprising
- providing an optical waveguide having a Fibre Bragg Grating;
- contacting the surface of the waveguide that is to be provided with a coating comprising a zeolite sensor material with a zeolite synthesis solution comprising a silicon source and a structure directing agent (such as a quaternary ammonium compound);
- depositing zeolite crystals from the zeolite synthesis solution on the surface of the waveguide, thereby forming a coating; and
- calcining the coating.

11. A method according to claim 10, wherein the zeolite is a non-acidic zeolite, preferably a non-acidic zeolite selected from the group of non-acidic MFI structure type zeolites, non-acidic LTA structure type zeolites, non-acidic MOR structure type zeolites and non-acidic FAU structure type zeolites, in particular a non-acidic zeolite selected from the group of silicalite, ZSM-5, Linde Type A, AlPO₄-5, zeolite X and zeolite Y, more in particular a non-acidic zeolite selected from the group of ZSM-5 and silicalite.

12. Method according to claim 9, wherein the surface of the waveguide is subjected to a functionalization treatment during which functional groups are provided on the surface, such as carboxylate groups, that can bind with metal ions for the MOF, thereafter contacting the surface, provided with the functional groups, with a metal-organic framework synthesis solution, and depositing a metal-organic framework from said synthesis solution to the surface of the waveguide.

13. Use of an optical sensor system according to any of the claims 1-5 for detecting a chemical substance, preferably a chemical substance selected from the group of alkanes, in particular an alkane having 1 to 12 carbon atoms, more in particular an alkane having 2 to 10 carbon atoms, more in particular a linear alkane having 2-10 carbon atoms, or wherein the chemical substance is an alkanol, an alkanone, or an ether.

14. Use according to claim 13, wherein the chemical substance is detected in a gaseous phase and/or wherein the chemical substance is detected at a temperature above 100 °C, in particular at a temperature in the range of 150-500 °C, more in particular at a temperature of 150-300 °C.

15. Use according to claim 13 or 14 , wherein the chemical substance is detected in a gas or oil reservoir.

## Patentansprüche

1. Optisches Sensorsystem zum Messen einer chemischen Substanz, wobei das Sensorsystem einen optischen Wellenleiter mit einem Faser-Bragg-Gitter umfasst, wobei der Wellenleiter mit einer Beschichtung versehen ist, umfassend ein nanoporöses Sensormaterial, die Sensoreinheit weiter umfassend eine optische Detektionseinheit zum Detektieren einer Änderung einer optischen Eigenschaft des Wellenleiters, wobei das Gitter im Kern des Wellenleiters vorhanden ist, die Beschichtung das Gitter mindestens im Wesentlichen umgibt, wobei die Beschichtung unter dem Einfluss der chemischen Substanz isotherm erweiterbar oder schrumpfbar ist, wodurch sie isotherm eine Änderung der axialen Dehnung des Gitters verursacht, wenn das Sensormaterial der chemischen Substanz ausgesetzt ist, wobei die Änderung durch die optischen Detektionseinheit detektierbar ist.

2. Optisches Sensorsystem nach Anspruch 1, wobei das Sensormaterial einen nicht-sauren Zeolith umfasst, wobei der nicht-saure Zeolith bevorzugt ausgewählt aus der Gruppe der nicht-sauren Zeolithe des Typs mit MFI-Struktur, nicht-sauren Zeolithe des Typs mit LTA-Struktur, nicht-sauren Zeolithe des Typs mit MOR-Struktur und nicht-sauren Zeolithe des Typs mit FAU-Struktur.

3. Optischer Sensor nach Anspruch 2, wobei der nicht saure Zeolith aus der Gruppe von Silicalit, ZSM-5, Linde Typ A, AlPO₄-5, AlPO₄-34, Zeolith X und Zeolith Y ausgewählt wird, bevorzugt ausgewählt aus der Gruppe von ZSM-5 und Silikalit.

4. Optischer Sensor nach einem der vorhergehenden Ansprüche, wobei das Sensormaterial ein metallorganisches Gerüst (MOF) umfasst, insbesondere ein MOF, dargestellt durch die Formel MₙOₖXᵢLₚ, wobei
- jedes M unabhängig ausgewählt ist aus der Gruppe der Metall- und Halbmetall-Ionen, insbesondere ausgewählt aus der Gruppe, bestehend aus Ti⁴⁺, Zr⁴⁺, Mn⁴⁺, Si⁴⁺, Al³⁺, Cr³⁺, V³⁺, Ga³⁺, In³⁺, Mn³⁺, Mn²⁺, Mg²⁺ und Kombinationen davon;
- n ist 1, 2, 3 oder 4, vorzugsweise 1 oder 3;
- k ist 0, 1, 2, 3 oder 4, vorzugsweise 0 oder 1;
- i ist 0, 1, 2, 3 oder 4, vorzugsweise 0 oder 1;
- p ist 1, 2, 3 oder 4, vorzugsweise 1 oder 3;
- 0 ist Sauerstoff
- jedes X ist unabhängig ausgewählt aus der Gruppe der Anionen, insbesondere aus der Gruppe einwertiger Anionen, weiter insbesondere aus der Gruppe bestehend aus OH⁻ Cl⁻, F⁻, I⁻, Br⁻, SO₄²⁻, NO₃₋, ClO₄₋ PF₆₋, BF₃₋-(COO)ₙ₋, R¹-(SO3)ₙ₋, R¹-PO₃)ₙ₋, wobei R¹ ausgewählt ist aus der Gruppe, bestehend aus Wasserstoff und Kohlenwasserstoffen, insbesondere Wasserstoff und C1-C12-Kohlenwasserstoffe, weiter insbesondere Wasserstoff und C1-C12-Alkyle, und wobei n 1, 2, 3 oder 4 ist;
- L ein Spacer-Ligand ist, insbesondere ein Spacer-Ligand ist, umfassend ein Radikal R, umfassend q Carboxylatgruppen (-COO⁻), wobei q 1, 2, 3, 4, 5 oder 6, vorzugsweise 2, 3 oder 4 ist, R kann insbesondere aus der Gruppe ausgewählt sein, bestehend aus C1-C12-Alkyl, C2-C12-Alken, C2-C12-Alkin, mono- und polycyclischem C6-C50-Aryl-, mono- und polycyclischem C3-C50-Heteroaryl- und organische Radikale, umfassend einen Metallstoff, ausgewählt aus der Gruppe bestehend aus Ferrocen, Porphyrin, Phthalocyanin und Schiffsche Base R^{x¹}R^{x²}-C = N-R^{x³}, wobei R^{X¹} und R^{X²} unabhängig voneinander aus der Gruppe ausgewählt sind, bestehend aus Wasserstoff, C1-C12-Alkyl, C2-C12-Alken, C2-C12-Alkin und mono- und polycyclischem C6-C50-Aryl und wobei RX3 ausgewählt aus der Gruppe, bestehend aus C1-C12-Alkyl, C2-C12-Alken, C2-C12-Alkin und mono- und polycyclischem C6- C50 Aryl.

5. Optisches Sensorsystem nach einem der vorhergehenden Ansprüche, wobei die Beschichtung eine Dicke von 1 bis 100 µm, bevorzugt von 3 bis 70 µm, bevorzugt von 5 bis 50 µm aufweist.

6. Optischer Wellenleiter mit einem Faser-Bragg-Gitter, wobei der Wellenleiter mit einer Beschichtung versehen ist, umfassend ein nanoporöses Sensormaterial, wobei das Gitter im Kern des Wellenleiters vorhanden ist, die Beschichtung das Gitter mindestens im Wesentlichen umgibt, wobei die Beschichtung unter dem Einfluss einer chemischen Substanz isotherm erweiterbar oder schrumpfbar ist, wobei dadurch isothermisch eine Änderung der axialen Dehnung im Gitter bewirkt wird, wenn das Sensormaterial der chemischen Substanz ausgesetzt wird, wobei diese Änderung durch eine optische Detektionseinheit detektierbar ist.

7. Wellenleiter nach Anspruch 6, wobei die Beschichtung einen nicht-sauren Zeolith umfasst, bevorzugt ausgewählt aus der Gruppe, ausgewählt aus der Gruppe der nicht-sauren Zeolithe des Typs mit MFI-Struktur, nicht-sauren Zeolithe des Typs mit LTA-Struktur, nicht-sauren Zeolithe des Typs mit MOR-Struktur und den nicht-sauren Zeolithe des Typs mit FAU-Struktur, insbesondere einen nicht-saurer Zeolith, ausgewählt aus der Gruppe von Silicalit, ZSM-5, Linde Typ A, AlPO₄₋5, Zeolith X und Zeolith Y, weiter insbesondere einen nicht-sauren Zeolith, ausgewählt aus der Gruppe von ZSM-5 und Silicalit.

8. Optischer Wellenleiter nach Anspruch 6 oder 7, wobei das Sensormaterial ein metallorganisches Gerüst (MOF) umfasst, insbesondere ein MOF, dargestellt durch die Formel MₙOₖXᵢLₚ, wie in Anspruch 4 definiert.

9. Verfahren zur Herstellung eines optischen Wellenleiters nach einem der Ansprüche 6 oder 7, das Verfahren umfassend das Vorsehen eines optischen Wellenleiters mit einem Faser-Bragg-Gitter und das Vorsehen einer Oberfläche des optischen Wellenleiters mit einer Beschichtung, umfassend das nanoporöse Sensormaterial.

10. Verfahren nach Anspruch 9, wobei das Sensormaterial einen Zeolith umfasst, das Verfahren umfassend
- Vorsehen eines optischen Wellenleiters mit einem Faser-Bragg-Gitter;
- Kontaktieren der Oberfläche des Wellenleiters, die mit einer Beschichtung zu versehen ist, umfassend ein Zeolithsensormaterial mit einer Zeolithsyntheselösung umfassend eine Siliziumquelle und ein Struktur lenkendes Mittel (wie z. B. quaternäre Ammoniumverbindung);
- Ablagerung von Zeolithkristallen aus der Zeolithsyntheselösung auf der Oberfläche des Wellenleiters, wodurch eine Beschichtung gebildet wird; und
- Kalzinieren der Beschichtung.

11. Verfahren nach Anspruch 10, wobei der Zeolith ein nicht-saurer Zeolith ist, bevorzugt ein nicht-saurer Zeolith, ausgewählt aus der Gruppe der nicht-sauren Zeolithe des Typs mit MFI-Struktur, nicht-sauren Zeolithe des Typs mit LTA-Struktur, nicht-sauren Zeolithe des Typs mit MOR-Struktur und nicht-sauren Zeolithe des Typs mit FAU-Struktur, insbesondere ein nicht-saurer Zeolith, ausgewählt aus der Gruppe von Silicalit, ZSM-5, Linde Typ A, AlPO₄₋5, Zeolith X und Zeolith Y, weiter insbesondere ein nicht-saurer Zeolith, ausgewählt aus der Gruppe von ZSM-5 und Silicalit.

12. Verfahren nach Anspruch 9, wobei die Oberfläche des Wellenleiters einer Funktionalisierungsbehandlung unterzogen wird, während der funktionelle Gruppen an der Oberfläche, wie Carboxylatgruppen, bereitgestellt werden, die sich mit Metallionen für das MOF verbinden können, danach Kontaktieren der Oberfläche, versehen mit den funktionellen Gruppen mit einer metallorganischen Gerüst-Syntheselösung und Ablagerung eines metallorganischen Gerüsts aus der Syntheselösung auf der Oberfläche des Wellenleiters.

13. Verwendung eines optischen Sensorsystems nach einem der Ansprüche 1 bis 5 zum Nachweis einer chemischen Substanz, bevorzugt einer chemischen Substanz, ausgewählt aus der Gruppe der Alkane, insbesondere eines Alkans mit 1 bis 12 Kohlenstoffatomen, weiter insbesondere eines Alkans mit 2 bis 10 Kohlenstoffatomen, weiter insbesondere eines linearen Alkans mit 2 bis 10 Kohlenstoffatomen, oder wobei die chemische Substanz ein Alkanol, ein Alkanon oder ein Ether ist.

14. Verwendung nach Anspruch 13, wobei die chemische Substanz in einer Gasphase detektiert wird und/oder wobei die chemische Substanz bei einer Temperatur über 100 °C, weiter insbesondere bei einer Temperatur im Bereich von 150 bis 500 °C, weiter insbesondere bei einer Temperatur von 150 bis 300 °C detektiert wird.

15. Verwendung nach Anspruch 13 oder 14, wobei die chemische Substanz in einem Gas- oder Ölreservoir detektiert wird.

## Revendications

1. Système de capteur optique pour mesurer une substance chimique, le système de capteur comprenant un guide d'ondes optique ayant un réseau de Bragg sur fibre, lequel guide d'ondes est pourvu d'un revêtement comprenant un matériau de capteur nanoporeux, l'unité de capteur comprenant en outre une unité de détection optique pour détecter une modification d'une propriété optique du guide d'ondes, dans lequel le réseau est présent dans le noyau du guide d'ondes, le revêtement entoure au moins sensiblement le réseau, lequel revêtement peut se dilater ou rétrécir de manière isotherme sous l'influence de la substance chimique, en provoquant ainsi de manière isotherme un changement de contrainte axiale dans le réseau lorsque le matériau de capteur est exposé à la substance chimique, lequel changement peut être détecté par l'unité de détection optique.

2. Système de capteur optique selon la revendication 1, dans lequel le matériau de capteur comprend une zéolite non acide, laquelle zéolite non acide est de préférence choisie dans le groupe comprenant des zéolites non acides du type à structure MFI, des zéolites non acides du type à structure LTA, des zéolithes non acides du type à structure MOR et des zéolithes non acides du type à structure FAU.

3. Capteur optique selon la revendication 2, dans lequel la zéolite non acide est choisie dans le groupe comprenant silicalite, ZSM-5, Linde de type A, AlPO₄-5, AlPO₄-34, zéolite X et zéolite Y, de préférence choisie dans le groupe comprenant ZSM-5 et silicalite.

4. Capteur optique selon l'une quelconque des revendications précédentes, dans lequel le matériau de capteur comprend un cadre organométallique (MOF), en particulier un MOF représenté par la formule MₙOₖXᵢLₚ, dans lequel
- chaque M est indépendamment choisi dans le groupe comprenant des ions métalliques et semi-métalliques, en particulier dans le groupe constitué de Ti⁴⁺, Zr⁴⁺, Mn⁴⁺, Si⁴⁺, Al³⁺, Cr³⁺, V³⁺, Ga³⁺, In³⁺, Mn³⁺, Mn²⁺, Mg²⁺, et de combinaisons de ceux-ci ;
- n est 1, 2, 3 ou 4, de préférence 1 ou 3 ;
- k est 0, 1, 2, 3 ou 4, de préférence 0 ou 1 ;
- i est 0, 1, 2, 3 ou 4, de préférence 0 ou 1 ;
- p est 1, 2, 3 ou 4, de préférence 1 ou 3 ;
- O est l'oxygène
- chaque X est choisi indépendamment dans le groupe comprenant des anions, en particulier dans le groupe comprenant des anions monovalents, plus particulièrement dans le groupe comprenant OH-Cl-, F-, I-, Br-, SO₄²-, NO₃-, ClO₄-, PF₆-, BF₃-, -(COO)ₙ-, R¹-(SO₃)ₙ-, R¹-PO₃)ₙ-, où R₁ est choisi dans le groupe comprenant de l'hydrogène et des hydrocarbures, en particulier de l'hydrogène et des hydrocarbures en C₁-C₁₂, plus particulièrement de l'hydrogène et les groupes alkyle en C₁-C₁₂, et où n est 1, 2, 3 ou 4 ;
- L est un ligand espaceur, en particulier un ligand espaceur comprenant un radical R comprenant q groupes carboxylate (-COO-), où q est 1, 2, 3, 4, 5 ou 6, de préférence 2, 3 ou 4. R peut en particulier être choisi dans le groupe comprenant alkyle en C₁-C₁₂, alcène en C₂-C₁₂, alcyne en C₂-C₁₂, aryle en C₆-C₅₀ mono et polycyclique, hétéroaryle en C₆-C₅₀ mono et polycyclique et des radicaux organiques comprenant un matériau métallique choisi dans le groupe comprenant le ferrocène, la porphyrine, la phtalocyanine et la base de Schiff R^{x1}R^{x2}-C = N-R^{X3}, où R^{X1} et R^{X2} étant choisis indépendamment dans le groupe comprenant hydrogène, alkyle en C₁-C₁₂, alcène en C₂-C₁₂, alcyne en C₂-C₁₂ et aryle en C₆-C₅₀ mono et polycyclique, et où RX3 est choisi dans le groupe comprenant alkyle en C₁-C₁₂, alcène en C₂-C₁₂, alcyne en C₂-C₁₂ et aryle en C₆-C₅₀ mono et polycyclique.

5. Système de capteur optique selon l'une quelconque des revendications précédentes, dans lequel le revêtement a une épaisseur de 1 à 100 µm, de préférence de 3 à 70 µm, de manière plus préférée de 5 à 50 µm.

6. Guide d'ondes optique comportant un réseau de fibres de Bragg, lequel guide d'ondes est pourvu d'un revêtement comprenant un matériau de capteur nanoporeux, dans lequel le réseau est présent dans le noyau du guide d'ondes, le revêtement entoure au moins sensiblement le réseau, lequel revêtement peut se dilater ou rétrécir de manière isotherme sous l'influence d'une substance chimique, en provoquant ainsi de manière isotherme un changement de contrainte axiale dans le réseau lorsque le matériau de capteur est exposé à la substance chimique, lequel changement peut être détecté par une unité de détection optique.

7. Guide d'ondes selon la revendication 6, dans lequel le revêtement comprend une zéolithe non acide, de préférence choisie dans le groupe comprenant des zéolites non acides du type à structure MFI, des zéolites non acides du type à structure LTA, des zéolithes non acides du type à structure MOR et des zéolithes non acides du type à structure FAU, en particulier une zéolite non acide choisie dans le groupe comprenant silicalite, ZSM-5, Linde de type A, AlPO₄-5, AlPO₄-34, zéolite X et zéolite Y, et plus particulièrement une zéolite non acide choisie dans le groupe comprenant ZSM-5 et silicalite.

8. Guide d'ondes optique selon la revendication 6 ou 7, dans lequel le matériau de capteur comprend un cadre organométallique (MOF), en particulier un MOF représenté par la formule MₙOₖXᵢLₚ telle que définie dans la revendication 4.

9. Procédé de fabrication d'un guide d'ondes optique selon l'une quelconque des revendications 6 ou 7, le procédé comprenant la fourniture d'un guide d'ondes optique ayant un réseau de Bragg sur fibre et la fourniture à une surface du guide d'ondes optique d'un revêtement comprenant le matériau de capteur nanoporeux.

10. Procédé selon la revendication 9, dans lequel le matériau de capteur comprend une zéolite, le procédé comprenant les étapes consistant à
- fournir un guide d'ondes optique ayant un réseau de Bragg sur fibre ;
- mettre en contact la surface du guide d'ondes devant être pourvue d'un revêtement comprenant un matériau de capteur de zéolite avec une solution de synthèse de zéolite comprenant une source de silicium et un agent de direction de structure (tel qu'un composé d'ammonium quaternaire) ;
- déposer des cristaux de zéolite provenant de la solution de synthèse de zéolite à la surface du guide d'ondes, en formant ainsi un revêtement ; et
- calciner le revêtement.

11. Procédé selon la revendication 10, dans lequel la zéolite est une zéolite non acide, de préférence une zéolithe non acide choisie dans le groupe comprenant des zéolites non acides du type à structure MFI, des zéolites non acides du type à structure LTA, des zéolithes non acides du type à structure MOR et des zéolithes non acides du type à structure FAU, en particulier une zéolite non acide choisie dans le groupe comprenant silicalite, ZSM-5, Linde de type A, AlPO₄-5, AlPO₄-34, zéolite X et zéolite Y, et plus particulièrement une zéolite non acide choisie dans le groupe comprenant ZSM-5 et silicalite.

12. Procédé selon la revendication 9, dans lequel la surface du guide d'ondes est soumise à un traitement de fonctionnalisation au cours duquel des groupes fonctionnels sont fournis sur la surface, tels que des groupes carboxylate, qui peuvent se lier à des ions métalliques pour le MOF, en mettant ensuite en contact la surface pourvue des groupes fonctionnels avec une solution de synthèse de structure organométallique et organique, et en déposant un cadre métallo-organique provenant de ladite solution de synthèse à la surface du guide d'ondes.

13. Utilisation d'un système de capteur optique selon l'une quelconque des revendications 1 à 5 pour détecter une substance chimique, de préférence une substance chimique choisie dans le groupe des alcanes, en particulier un alcane ayant 1 à 12 atomes de carbone, plus particulièrement un alcane ayant de 2 à 10 atomes de carbone, plus particulièrement un alcane linéaire ayant de 2 à 10 atomes de carbone, ou dans laquelle la substance chimique est un alcanol, une alcanone ou un éther.

14. Utilisation selon la revendication 13, dans laquelle la substance chimique est détectée dans une phase gazeuse et/ou dans laquelle la substance chimique est détectée à une température supérieure à 100°C, en particulier à une température dans la plage de 150 à 500°C. plus particulièrement à une température de 150 à 300°C.

15. Utilisation selon la revendication 13 ou 14, dans laquelle la substance chimique est détectée dans un réservoir de gaz ou de pétrole.
